Europäisches Patentamt

**European Patent Office**     (11) Publication number: **0 135 840**
**A2**

Office européen des brevets

## EUROPEAN PATENT APPLICATION

(21) Application number: **84110306.2**

(22) Date of filing: **29.08.84**

(51) Int. Cl.⁴: **A 61 B 5/00,** A 61 B 5/04, G 01 N 21/27

(30) Priority: **30.08.83 US 527726**
**24.08.84 US 644051**

(43) Date of publication of application: **03.04.85**
**Bulletin 85/14**

(84) Designated Contracting States: **AT BE CH DE FR GB IT LI LU NL SE**

(71) Applicant: **NELLCOR INCORPORATED, 25495 White Sell Street, Hayward California 94545 (US)**

(72) Inventor: **Corenman, James E., 1095 Sherman Avenue, Menlo Park California 94025 (US)**
Inventor: **New, William, Jr., 95 Skywood Way, Woodside California 94062 (US)**
Inventor: **Goodman, David E., 454 Roosevelt Way, San Francisco California 94114 (US)**
Inventor: **Yelderman, Mark, 736 La Mesa, Menlo Park California 94025 (US)**

(74) Representative: **Vossius Vossius Tauchner Heunemann Rauh, Siebertstrasse 4 P.O. Box 86 07 67, D-8000 München 86 (DE)**

(54) **Perinatal oximeter.**

(57) An intravaginal and intrauterine sensor for transillumination of a blood-perfused portion of fetal tissue to measure light extinction. The sensor comprises at least one light source (21, 23) and at least one photo-sensor (20, 22) with a shielding (30). The sensor may be mounted onto a paddle-shaped probe provided with signal connections (25, 26) contained in an insulated cable (27) leading to a measurement device. Alternatively, the sensor may include an opaque suction cup or other attaching apparatus for disposition on a protruding portion of the fetus. Most preferably, the sensor includes a housing.

- 1 -

VOSSIUS · VOSSIUS · TAUCHNER · HEUNEMANN · RAUH
PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our ref.: T 262 EP
Case: NEL-4 (EPO)
NELLCOR INCORPORATED
Hayward, U.S.A.

August 29, 1984

PERINATAL OXIMETER

## Background Of The Invention

This invention relates to a sensor for use on a fetus during birth. Specifically, this sensor measures arterial oxygen saturation, pulse amplitude, pulse rate, rhythm, electrocardiogram and temperature using non-invasive photoelectric determination.

For many years, physicians have non-invasively monitored the fetal heartbeat acoustically, through the mother's abdomen, using a stethoscope. Often, however, this listening apparatus is inadequate to receive weak sound signals from the fetal heart, especially when that fetal heartbeat is masked by maternal sounds.

With the advent of electronics, physicians have monitored fetal heartbeat electrically using non-invasive electrocardiogram (EKG) electrodes attached to the mother's lower abdomen. Unfortunately, the fetal EKG received is a weak signal and often is masked or confused by the electrical activity of the mother's heartbeat. Improvement can be obtained by attaching a scalp electrode to the fetus to obtain a good fetal EKG (see below).

-2-

In recent years, physicians have monitored fetal heartbeat ultrasonically using the Doppler effect ultrasound technique. This non-invasive technique bounces high-frequency soundwaves off the beating fetal heart through the mother's abdomen to measure heartbeat. The Doppler technique typically provides an acoustic output of the fetal heartbeat.

All the above devices (stethoscope, EKG, Doppler) monitor only the rate and rhythm of fetal heartbeat. All techniques which assess fetal heart rate detect fetal distress by changes in fetal heart rate. Abnormal changes in fetal heart rate reflect but may not be pathognomonic for fetal hypoxia.

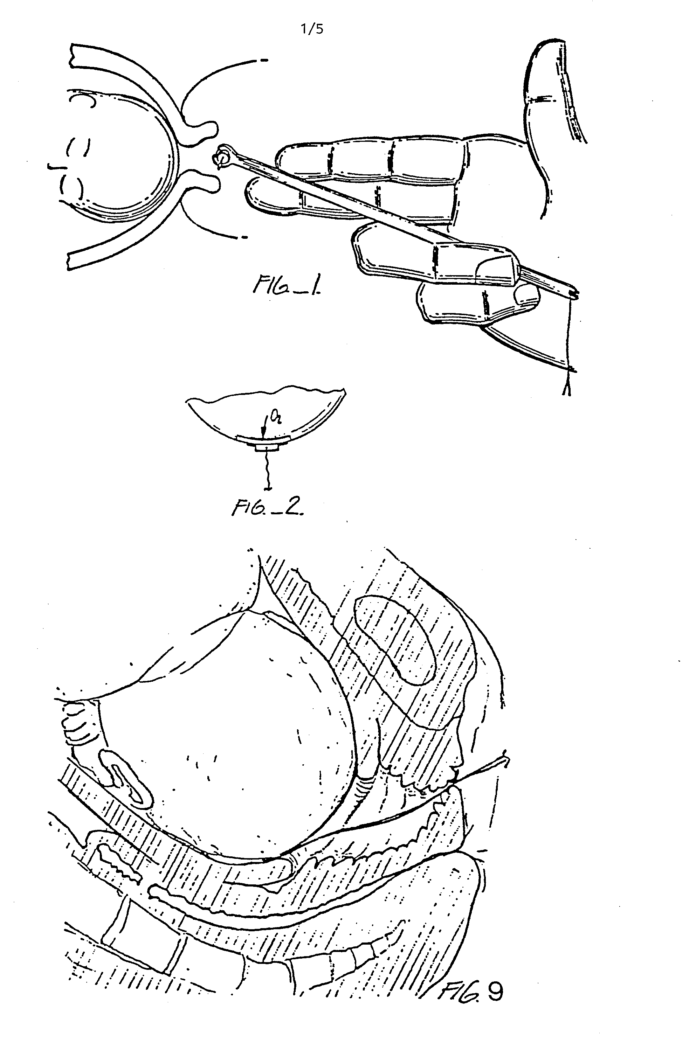

One representative technique for following fetal heart rate involves a fetal scalp cutaneous EKG electrode device that screws into the fetal scalp (see Fig. 1). A vaginal probe is introduced onto the fetal scalp after the membranes have ruptured. Fetal EKG can be detected and recorded. Fetal well being can be ascertained by changes in fetal heart rate concomitant with uterine contractions. Unusually low fetal heart rates or unusually high fetal heart rates may be indicative of fetal distress. However, even in the best trained clinicians the ability to assess definite fetal distress from fetal heart rate pattern is restricted and limited.

Monitoring fetal oxygen is an effective way to detect and treat hypoxia in the fetus during labor. Hypoxia is a condition of oxygen deprivation which may occur undetected during birth resulting in brain damage (viz. cerebral palsy) and other vital organ deterioration.

In an uneventful birth, a fetus suffers modest oxygen deprivation during uterine contractions but recovers an adequate oxygen level between contractions. During prolonged oxygen deprivation, the metabolism of the fetus is changed to a non-oxygen-requiring (anaerobic) process that produces lactic acid. This anaerobic change effects depression of the pH level of the fetal tissue from alkaline to acid.  In order to prevent fetal damage from hypoxia, physicians have monitored fetal oxygen levels indirectly by measuring the tissue and/or blood pH level, which indeed becomes substantially acidic after hypoxia persists for several minutes.  In one such method, a fetal scalp cutaneous pH electrode device similar to the fetal cutaneous EKG electrode device (see Fig. 1) is used in monitoring fetal pH.

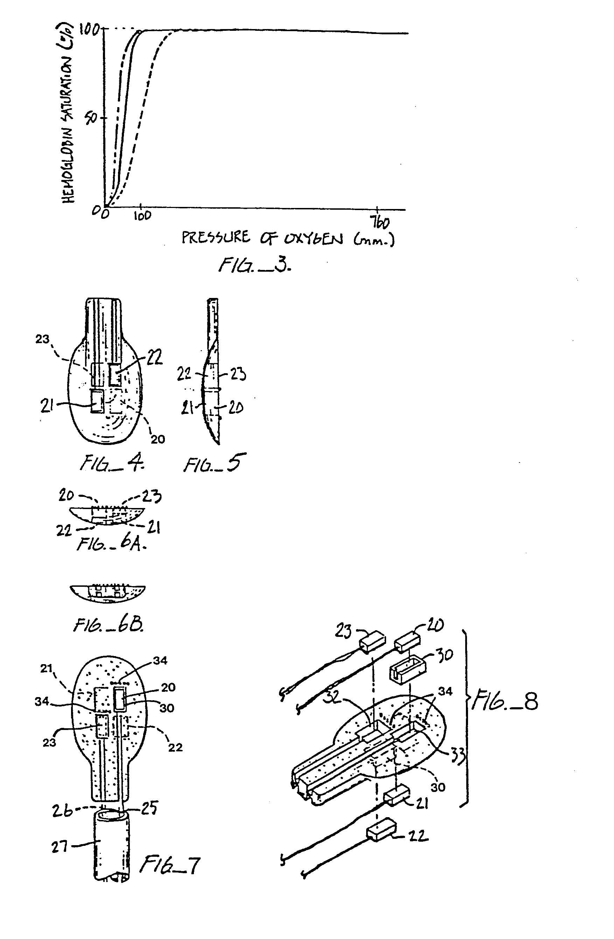

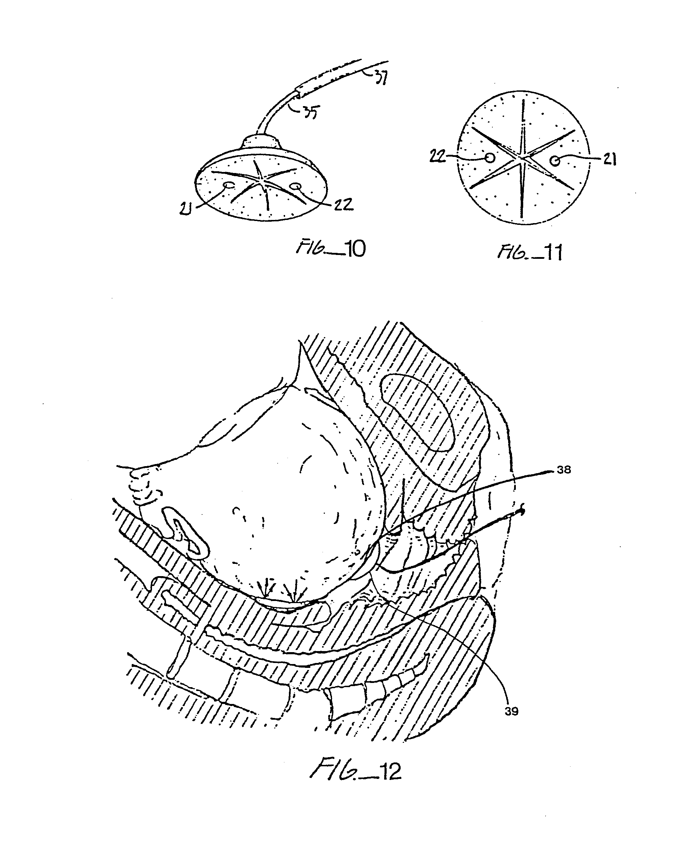

Another technique for following fetal oxygen level involves the Clark electrode (see Figs. 2 and 3).  This technique requires an exposed, relatively accessible presenting portion of fetal tissue through the dilated cervix, upon which an airtight dam is adhesively fixed.  The airtight dam serves to capture oxygen as it diffuses through the fetal cutaneous layer.  The accumulated diffused oxygen is electrochemically measured by the Clark electrode to provide an indication of oxygen in the fetal tissue. This technique suffers from the slowness of oxygen diffusion from the arterial blood stream out to the cutaneous layer.  Further this technique suffers from significant incidence of imperfect air seal or an interruption of air seal that renders measurement errant and thus without utility.

The clinical problem answered by the invention disclosed herein is the need of physicians for a non-invasive continuous fetal monitor that conveniently and safely provides:

1)    direct measurement of fetal oxygen level;

2)    instant indication of the adequacy of the fetal tissue perfusion for transporting oxygen through the fetus' body; and

3) means of monitoring the rate and rhythm of the fetal heartbeat similar to that offered by conventional electrical and acoustical fetal monitors.

The objective is to provide sufficiently early warning of fetal distress such that appropriate alteration or initiation of treatment (e.g., administration of intravenous fluids and drugs, Caesarian section surgery) can prevent the tragedy of cerebral palsy or other hypoxic organ damage.

## Summary Of The Invention

An intrauterine sensor for transillumination of a blood-perfused portion of fetal tissue to measure light extinction is disclosed. The sensor may be mounted upon a paddle-shaped probe provided with signal connections contained in an insulated cable leading to a measurement device. The sensor can be fastened by surgical glue or adhesive material or a cutaneous screw attachment for disposition on a portion of the fetus accessible through the dilated cervix.

Most preferably, however, the sensor includes a housing having an upper portion and a lower portion. A light source for transilluminating the fetal tissue is contained in the upper portion. The lower portion includes a cavity which is open at one end adapted for placement against the fetal tissue. A light sensor for receiving the light having transilluminated the fetal tissue is mounted in the cavity. For temporarily securing the sensor to a presenting portion of fetal tissue, attachment means communicate with the cavity for connecting the sensor to a vacuum source to provide a suction force between the sensor and the fetal presenting portion when the open end of the cavity is placed against the fetal presenting portion. Attachment can also be facilitated by the use of medical glue or other adhesive as an adjunct to the vacuum.

If the paddle-shaped probe is used, at least one light source is mounted on the first side which lies against the fetal tissue in such a manner as to illuminate the fetal tissue. At least one photo-sensor is mounted on the same first side to receive the light after it has passed through or been reflected from the fetal tissue.

If the suction cup is used, at least one light source is mounted to the first portion of the concavity and at least one photo-sensor is mounted to the second portion of the same concavity.

If the cutaneous screw attachment is used, at least one light source is mounted to the first portion of a thin cylindrical, blunt-ended probe. At least one photo-sensor is mounted on the same first portion of the probe to receive light after it has passed from the light source through the fetal tissue.

If the surgical glue is used, at least one light source is mounted to the first portion of a probe. At least one photo-sensor is mounted on the same first portion of the probe to receive light after it has passed from the light source through the fetal tissue.

With all designs, light is transmitted from the light source to the photo-sensor through blood perfused tissue of the fetus. Pulsations of fetal blood cause modulation of the light transmission through the fetal tissue. The modulated output signal of the photo-sensor is transmitted through the insulated cable of the measurement device.

With all designs, the apparatus is introduced into the vagina after the membranes have ruptured. The apparatus may be moved up the vagina using semi-flexible columnar tubing, through the dilated cervix, for disposition on a presenting portion of blood-perfused fetal tissue.  Each device
remains adherent to that cutaneous layer during birth
until delivery is complete.

Measurement from the modulated output of
the photo-sensor yields information about the pulsating blood supply to the transilluminated fetal tissue,
including but not limited to:  oxygen saturation of
the hemoglobin in the blood; the volume of individual
blood pulsations supplying the flesh; and the rate
of blood pulsations occurring with each fetal heartbeat.

The apparatus may also include, in addition
to the aforementioned light sources and sensors,
means for measuring heart electrical activity on the
fetus and means for measuring the temperature of the
fetus.

The apparatus may include, in addition to
the aforementioned fetal sensing means, a similar
means for measuring heart electrical actvity, blood
pulsation and oxygen saturation in the overlying
maternal tissue.

An object of this invention is to provide
an apparatus with light source and a photo-sensor
for transillumminating a blood-perfused portion of
the fetus.  The light source and the photo-sensor
are mounted on a probe.  This probe and its attaching
signal transmission cable are introduced through the
vagina and attached to a presenting portion of the
fetus.  The probe conforms closely and non-invasively
to the fetal cutaneous layer.

An advantage of the apparatus closely bonding to the fetal cutaneous layer is that measurement
distortion, commonly caused by motion artifact, is
minimized.  The suppression of motion artifact renders
measurement more accurate than a sensor with higher
mass and aspect ratio.

A further advantage of this invention is that the probe is attached superficially to the fetal head and neither invades nor occludes the blood flow to be interrogated.

A further advantage of the disclosed invention is that the mother's hearbeat may simultaneously be measured and easily be distinguished from the fetal heartbeat whereby confusion of measurement resulting from erroneous placement of the probe is immediately apparent and will not cause error.

A further advantage of the disclosed invention is that the same vital signs and measurements as are taken of the fetus may be simultaneously taken of the mother. The probe may be provided with two sensor sets: one facing the mother's body, the other facing the fetus.

A further advantage of the disclosed invention is that the resultant direct and continuous measurements of the fetal arterial oxygen saturation afford the physician the opportunity to diagnose and take immediate responsive action and corrective therapy. For instance, unlike the prior art which had a limited capability to make delayed, indirect measurement of oxygen deprivation already having deleterious effects upon the fetus, the disclosed invention will allow the physician to know immediately when a fetus is initially oxygen deprived. This allows the physician to take steps to prevent the deterioration of brain and other vital organs.

A further advantage of the disclosed invention is that the temperature of the area of the fetus may be taken simultaneously with the recording of the arterial oxygen saturation.

A further advantage of the disclosed invention is that a patient's mechanical blood pumping activity (pulse) and oxygen saturation may be instantaneously recorded and compared for diagnosis and early treatment.

A further advantage of the disclosed invention is that a physician may compare simultaneous instantaneous readings from both the fetus and the mother of their individual heart rate, electrocardiogram, oxygen saturation, and pulse amplitude.

A further advantage is that the disclosed invention accurately measures arterial saturation even in circumstances when the partial pressure of oxygen is extremely low. A fetus receives oxygen from the maternal circulation, the oxygen diffusing from the mother's blood through the placenta into the fetal blood. The prior art Clark electrode has a high incidence of inaccurate (i.e. excessively low) oxygen saturation estimation when the partial pressure of the oxygen is low. The disclosed invention does not suffer from this handicap.

A further advantage is the complete absence of erroneous measurement by the disclosed invention due to an imperfect seal between the sensor and the fetus. The prior art relied for accurate measurement upon creating and maintaining an absolutely perfect airtight seal between the apparatus and the fetus. Any leak resulted not only in erroneous measurement but the deceptive measurement could lead the physician to conclude in error that the fetus was receiving sufficient oxygen when it was not.

A further advantage is the elimination of the risk of brain damage, eye damage or serious infection in the fetus. The sharp corkscrew pH or EKG electrode physically penetrates the presenting portion of cutaneous fetal tissue. Upon occasion that presenting portion is the fetal eye or fontanel (the soft bone-free midscalp spot over the brain). Whenever the fontanel or an eyelid is pierced, additional complications can occur. In approximately 5% of the cases where the delicate skin of the fetus is broken, infections do occur. The disclosed invention does not expose the fetus to infection risk from skin penetration.

A further advantage of the disclosed invention is that the parameters indicative of fetal condition are measured without the fetal head hair obstructing true readings. The prior art, the Clark electrode in particular, required a fetal presenting portion offering an airtight seal. It is very common for a fetus to have hair which presents a rough surface conducive to leakage. The prior art resolved this by shaving the hair until sufficient fetal head portion is exposed which presented delay and risk of infection should the scraping and shaving of the head puncture the skin. The disclosed invention measures accurately without regard to the texture of the fetal presenting portion.

A further advantage of the disclosed invention is the ability to measure fetal arterial oxygen saturation directly rather than indirectly. The ability to directly and instantly measure blood oxygen content will accelerate positive diagnosis and appropriate treatment of fetal hypoxia. This is in contrast to the prior art where the fetal arterial oxygen level could only be measured by monitoring indirect effects (e.g., acidosis) of deteriorative changes already in progress. The invention disclosed herein gives direct measurement of fetal arterial oxygen saturation.

A further object of this invention is to provide a combination of sensors mounted on a single probe to provide simultaneous and continuous fetal monitoring of several parameters, including but not limited to arterial hemoglobin oxygen saturation, pulse amplitude, pulse rate and rhythm, fetal electrocardiogram and fetal body temperature. The simultaneous and continuous availability of multiple important physiologic paraments provides instant means to
cross-validate and corroborate measurements obtained
with this probe, thereby improving substantially the
clinical utility of the obstetric monitor herein
disclosed compared to traditional single parameter
(acoustic, electric or pH) monitors.

A further object of this invention is to
provide a method for making a form-fitting sensor.
In the assembly of this invention, the light source
and photo-sensor are mounted to thin substrates,
being of such small dimension as to tightly conform
to the probe surface with low aspect ratio. There
results a simple flat flexible opaque probe apparatus
which is in conformance to the cutaneous cranial
area being interrogated for arterial saturation.

A further advantage of this invention is
that it is fully sterilizable and low in cost.

In summary, the resultant apparatus is
sanitary, non-invasive, in full conformance to the
skin and provides accurate measurement of heartbeat,
arterial oxygen saturation and other parameters in
both fetus and mother.

### Brief Description of Drawings

Other objects, features and advantages of
this invention will become more apparent after referring to the following drawings in which:

Fig. 1 is a perspective illustration of a
prior art pH or EKG electrode that punctures the
fetal cutaneous layer.

Fig. 2 is a graphical representation of
the prior art Clark electrode which attaches to the
fetal presenting portion and requires an airtight seal to yield a measurement of oxygen partial pressure, reflecting the amount of collected diffused oxygen in the airtight cell.

Fig. 3 is a graph representing the oxyhemoglobin association curve presenting oxygen saturation of hemoglobin in relation to the partial pressure of oxygen present.

Fig. 4 is a perspective illustration of one side of a paddle-shaped probe in accordance with the present invention.

Fig. 5 is a side view of the paddle-shaped probe of Fig. 4.

Figs. 6A and 6B are illustrations of the paddle-shaped probe of Fig. 4 viewed head-on from each end showing the paired photoelectric components mounted in place and gripping bumps.

Fig. 7 is a perspective representation of another side of the paddle-shaped probe showing the light source and light sensor affixed in apertures.

Fig. 8 is a perspective exploded illustration of the paddle-shaped probe showing a combination of sensors in order and location of assembly.

Fig. 9 is a perspective illustration of the paddle-shaped probe in place anatomically.

Fig. 10 is a perspective illustration of an alternative embodiment of the invention showing a suction cup probe and signal transmission cable.

Fig. 11 is an isometric view of the concavity of the suction cup probe of Fig. 10.

Fig. 12 is a perspective illustration of the suction cup probe in place anatomically.

Fig. 13 is a perspective illustration of an alternative embodiment of the invention wherein the probe is held in place by a vacuum.

Fig. 14 is a bottom plan view of the vacuum probe of Fig. 13.

-12-

Fig. 15 is a partial section view showing the vacuum probe held in place against the fetal tissue, taken along lines 15-15 in Fig. 14.

Fig. 16 is a section view showing the vacuum probe held in place against the fetal tissue, taken along lines 16-16 in Fig. 14.

## Description of The Preferred Embodiments

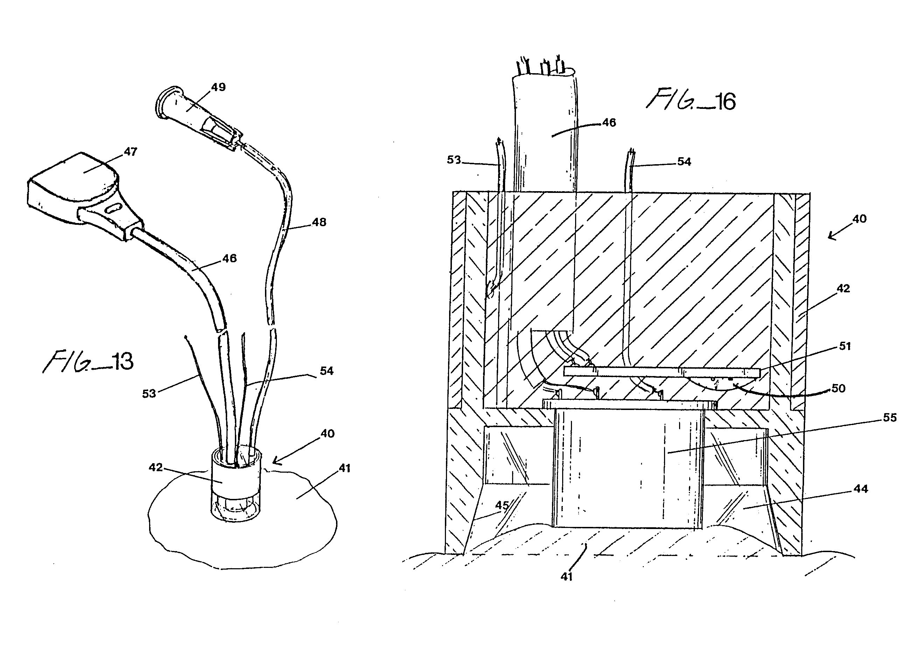

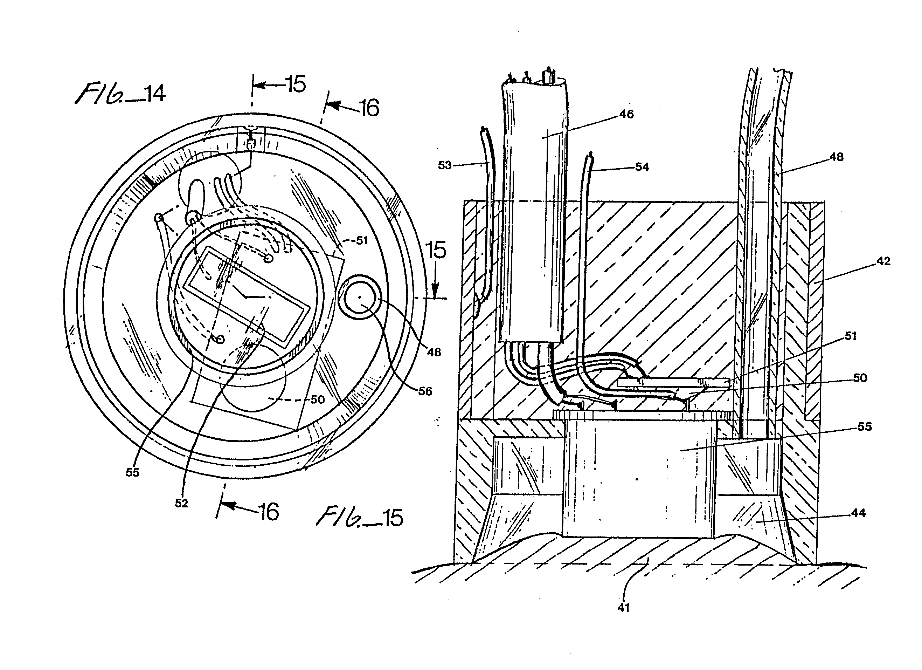

As is shown in Fig. 4, a small, paddle-shaped probe is provided with four apertures, two on either side, for reposit of two sets of paired electrical components. A suitable substance for the probe composition that is commecially available is sold under the trademark SILASTIC, by Dow Corning. This is a moldable, translucent, silicon rubber which acts as a spatially diffused light source when in contact with an illuminated light-emitting diode. At least one light source 21 or 23, and at least one photo-sensor 20 or 22, the photo-sensors having shielding 30, 32, are located respectively in the two apertures on each side as shown (see Fig. 8). The exploded view depicted in Fig. 8 illustrates the resulting assembly of one light source 21 and a photo-sensor 22 mounted to one side of the elongated paddle with another light source 23 and another photo-sensor 20 preferably mounted to the other side of the paddle-shaped probe. As is best shown in Figs. 7 and 8, the paddle side making contact with the fetus is provided with two rows of gripping bumps 34 for traction on the fetal skin and to aid in separating any hair thereon to expose the fetal skin.

The light sources 21, 23 are preferably light-emitting diodes. A light-emitting diode that is suitable and commercially available is manufactured by General Instrument Company with a typical wavelength between 650 and 950 nanometers. Each light-emitting diode 21, 23 is mounted on a ceramic substrate by gluing, with electrical connections provided by wire bonding. The light sources 21, 23 are each recessed into an aperture 32 (see Fig. 8), fastened to the paddle-shaped probe such that the light-emitting diode conforms to the planar surface of the probe.

The photo-sensors 20, 22 shown each include a ceramic substrate electrical connections and a light sensitive surface fastened to the ceramic substrate by gluing and by wire bonding. The photo-sensors 20, 22 are recessed into the apertures 33 and are fastened to the paddle-shaped probe, such that the photo-sensors conform to the planar surface of the probe (Figs. 6A, 6B).

For reference, each of the ceramic substrates shown in Figs. 8 (i.e., 20, 21, 22, 23) is 4mm x 6mm or of such other appropriately small dimensions.

Electrical connections 25, 26 shown in Fig. 7 are provided leading from the probe, down detachable columnar tubing 27, which tubing aids in placing the probe up the vagina to the fetus, to a measuring device. An electrical connection that is suitable and commercially available is silicone rubber coated shielded multiconductor miniature cable.

The mounting of the photo-sensors 20, 22 and the light sources (light-emitting diodes) 21, 23 onto the substrates herein disclosed can be accomplished through other configurations. For instance, an integrated chip or a thin film construction may be desirable for mass production of either the light source or photo-sensor. Fiber optic connection between the substrates and the measuring device is also suitable.

As shown in Figs. 6A and 6B, the paddle-shaped probe has planar conformity wide enough to incorporate the two sets of paired photoelectric

0135840

-14-

components 20, 21, 22, 23. Suitable dimensions of the paddle-shaped probe are 15mm x 15mm.

As shown in Fig. 6A, the two sets of paired photo-sensors 20, 22, preferably photo-diodes, and light sources 21, 23 are mounted into the probe facing off opposite sides of the transparent paddle-shaped probe. The light source is preferably a 5mm distance away from the photo-sensor.

As shown in Fig. 8, the photo-diodes 20, 22 are encased in metal shielding shaped into boxes 30, with edges extending into the paddle at each corner. The shielding is composed of silver or some other composite being an electrical conductor. This shielding serves the dual purpose of excluding ambient light and of providing a metal surface suitable for an electrocardiogram sensor, i.e., by serving as electrodes connected to EKG equipment by electrical leads (not shown).

As generally illustrated by Fig. 8, the resulting probe may be provided with a combination of sensors including, but not limited to, two arterial oxygen sensors, two electrocardiogram sensors, and one thermistor (not shown). Using appropriate techniques, the electrical signals provided by the arterial oxygen sensors can be used to determine fetal and material pulse rate, rhythm and amplitude, as well as oxygen level in the blood.

Alternatively, as shown in Fig. 10, a small suction cup is provided with one set of photoelectric components: a light source 22 and a photo-sensor 21 mounted on the same concave side of the cup. Referring generally to Fig. 11, two ceramic substrate portions are indicated. To one ceramic substrate, a photo-sensor 22 is mounted. To the other ceramic substrate, a light source 22 is mounted.

As discussed above, the light source 22 may be a light-emitting diode mounted to a ceramic substrate by gluing and by wire bonding. This light-emitting diode 22 conforms to a thin planar layer which is fastened to the concave side of the cup.

The photo-sensor 21 shown includes a ceramic substrate, electrical connections and a light sensitive surface fastened to the ceramic substrate by gluing and by wire bonding. This photo-sensor 21 conforms to the planar layer of the concavity. For reference, each of the two ceramic substrates is 4mm x 6mm or other appropriately small dimensions. Electrical connections 35 are provided leading from the probe, down the columnar tubing 37 (Fig. 10). The mounting of the photo-sensor 21 and the light-emitting diode 20 onto the substrates herein disclosed can be accomplished through other configurations. For instance, an integrated chip or a thin film construction may be desirable for mass production of either the light source or photo-sensor. Fiber optic connection between the substrates and the measuring device is also suitable.

Referring to Fig. 12, the probe with suction cup 39 is shown placed cutaneously on the fetal presenting portion 38. The suction cup is seated on the presenting portion of the fetus 38, and as pressure is exerted from the columnar tubing toward the fetal head air is expelled from the top of the concavity of the cup, at which point the tissue moves to fill the small space where air is expelled creating a suction between the cutaneous layer and the cup.

For measuring blood oxygen level, arterial oxygen sensors (i.e., two sets of photo-sensors and light sources) may be provided on either or both sides of the paddle. In such an arrangement, the light sources and photo-sensors on each side would preferably be adapted to operate at different wavelengths, in accordance with techniques for measuring the oxygen saturation.

An alternative and most preferred embodiment of the invention which uses a low-level vacuum to secure the sensor to the fetal tissue is illustrated in Figs. 13-16.

Referring first to Fig. 13, the probe or sensor 40 is shown held in place against fetal presenting portion 41. Suction tube 48 connected to the sensor terminates in fixture 49 for connecting the sensor to a suction source (not shown) such as those which are typically available in hospitals. Likewise, electrical cable 46 attached to the sensor terminates in a connecting member 47 for connecting the sensor to electrical measuring equipment. Electrical cables 53, 54 provide attachment means to an EKG monitor, as described below.

The sensor comprises a relatively small, semi-hollow cylinder made from an optically transparent material such as LEXAN polycarbonate or SILASTIC silicone rubber. (LEXAN and SILASTIC are trademarks of DuPont and Dow Corning, respectively). Materials such as SILASTIC silicone rubber, being relatively soft, attach more gently to the fetal skin. Referring to Figs. 14-16, a stainless steel electrode 42 formed around the outside of the upper portion of the sensor, as shown, contacts the vaginal and uterine walls and serves a reference electrode for the fetal EKG. Electrical cable 53 connects the electrode 42 to the EKG monitoring equipment, as mentioned above.

The optical components of the sensor comprise a light source 50, preferably a light-emitting diode, mounted on substrate 51, and a light sensor 52, preferably a photo-diode (see Fig. 16). The photo-diode is mounted in a stainless steel can 55, which is mounted concentrically in the sensor housing 43, as best shown in Figs. 14 and 15. Can 55 extends downwardly into a hollow space or cavity 44 formed in the lower sensor housing. This cavity is open at
one end, as shown, for placement against the fetal
skin (Figs. 15, 16). With the sensor in place, can 55
contacts the fetal tissue and serves as an electrode
for the fetal EKG. Electrical cable 54 connects
this electrode 55 to be EKG monitoring equipment, as
mentioned above.

As can best be seen in Fig. 14, the photo-
diode 52 is mounted such that it is spaced from the
inner surface of the sensor, a distance which is
preferably 0.1". In this configuration, the substrate-mounted light source, light-emitting diode 50,
is positioned directly above the light sensor, photo-
diode 52. This reduces the possibility of undesirable interference or cross-talk caused by extraneous
light originating from the light source reaching the
light sensor without passing through the fetal flesh.
The sensor is potted in an optically clear material
such as medical grade epoxy or silicone rubber.

Other components of the sensor mentioned
in passing above include electrical cable 46 for
attachment of the sensor to equipment which permits
measurement of the oxygen level in the fetal blood
and vacuum tube 48 for connecting the sensor to a
suction source (not shown). Suction tube 48 is
channeled through the upper portion of the sensor
housing 43 and is ported to the interior cavity 44
of the sensor through opening 56 (see Figs. 14, 15).
The sensor is also provided with a beveled edge 45
around the inner diameter of the cavity opening, to
facilitate sealing when the vacuum is applied.

In use during childbirth, once the membranes have ruptured the sensor can be manually
placed on the fetal presenting part 41, usually the
head, through the vagina (see Fig. 16) and a low
level vacuum applied through fixture 49 and suction
tube 48. The skin of the fetal presenting part is sucked up around the sensor, as shown, to form an optical barrier substantially preventing ambient light from entering the interior portion of the sensor. This assures that the photo-diode 50 contained in the can 55 receives light which has passed through fetal tissue only.

Applying a vacuum to the sensor causes the stainless steel photo-diode can 55 to press against and establish electrical contact with the fetal skin, as shown in Fig. 16, permitting the can to be used as the fetal EKG electrode in the manner described generally above.

The structure and arrangement of the sensor is such that when the light source is operating, the entire sensor functions in the manner of a light-transmissive pipe, assuring that an adequate level of light is transmitted to the fetal skin despite the presence of hair, blood, mucous or other substances which tend to decrease the light level.

As discussed above in connection with the description of the paddle-shaped device, for measuring blood oxygen saturation, the sensor may be provided with two light sources, both located in the upper portion of the sensor 40 as in Fig. 16, and two light sensors, both located in can 55. In such an arrangement, the light source-light sensor pairs would preferably operate at different wavelengths.

Above is disclosed an intravaginal and intrauterine sensor for transillumination of a blood-perfused portion of fetal tissue to measure light extinction. The sensor may be mounted onto a paddle-shaped probe provided with signal connections contained in an insulated cable leading to a measurement device. Alternatively, the sensor may include an opaque suction cup or other attaching apparatus for disposition on a protruding portion of the fetus. Most preferably, the sensor includes a housing having an upper portion and a lower portion. The lower portion includes a cavity which is open at one end for placement against the fetal tissue. Attachment means communicate with the cavity for connecting the sensor to a vacuum source to provide a suction force to secure the sensor against the fetal tissue.

For the paddle-shaped probe, a light source is mounted on the first side of the probe that is designated to be placed against the fetal tissue in such a manner as to illuminate said tissue. A photo-sensor is mounted on same first side in such a manner as to receive said illumination after the light has passed through said fetal tissue. For the suction cup probe, a light source is mounted to the first side of the concavity of the suction cup and a photo-sensor is mounted to the opposite side of the same concavity. With the vaccum sensor, a light source is mounted in the upper housing portion and a light sensor is mounted in the cavity. The housing is optically transmissive and adapted to provide an optical path between the light source and the fetal tissue.

CLAIMS:

1.   A sensor for non-invasive uterine insertion to measure light extinction during transillumination of a presenting portion of blood-perfused fetal tissue, comprising:

a light-conductive housing having an upper portion and lower portion, said lower portion including a cavity open at one end adapted for placement against said fetal presenting portion;

a light source in said upper housing portion for transilluminating said fetal presenting portion;

a light sensor mounted in said cavity for receiving light having transilluminated said fetal presenting portion; and

attachment means communicating with said cavity for connecting said sensor to a vacuum source for temporarily securing the sensor to said fetal presenting portion by providing a suction force between said sensor and said fetal presenting portion when the open end of said cavity is placed against the fetal presenting portion.

2.   The sensor of claim 1, further comprising a pair of electrocardiogram electrodes,

the first of said electrodes being affixed to the outside of said sensor housing for electrically contacting the uterus when the sensor is attached to the fetus, and

the second of said electrodes being positioned in said cavity for electrically contacting the fetal presenting portion when the sensor is attached to the fetus.

3.   The sensor of claim 1 or 2, further comprising shielding means for obstructing light not transilluminating said fetal presenting portion from reaching the light sensor.

4.  The sensor of claim 3, wherein said shielding means is also an electrocardiogram electrode.

5.  The sensor of any of claims 1 to 4, wherein said light source is positioned in the upper housing portion substantially above said light sensor in the cavity.

6.  The sensor of any of claims 1 to 5, wherein said light source is substrate mounted.

7.  The sensor of any of claims 1 to 6, wherein said housing is optically transmissive and is adapted for providing an optical path conducting light from said light source to said fetal presenting portion.

8.  The sensor of any of claims 1 to 7, wherein the open end of said cavity is provided with a beveled edge.

9.  The sensor of any of claims 1 to 8, wherein an adhesive is provided to aid in securing the sensor to said fetal presenting portion.

10.  The sensor of any of claims 1 to 9, wherein a second light source is provided in said upper housing and a second light sensor is provided in said cavity.

11.  An apparatus for non-invasive uterine insertion to measure light extinction during transillumination of a portion of blood-perfused fetal tissue, comprising:

an introducing and orienting probe having a surface for fetal cutaneous conformance;

a light source mounted on said probe surface to emit light to and transilluminate said fetal tissue;

a light sensor mounted on said probe surface to receive light having transilluminated said fetal tissue; and

a shielding device associated with said light sensor for preventing light not transilluminating said fetal tissue portion from reaching the light sensor.

12.    The apparatus of claim 11, wherein said shielding is a close-fitting box substantially enclosing said light sensor.

13.    The apparatus of claim 11 or 12, wherein said shielding is electrically conductive and is further adapted to provide an electrocardiogram sensor.

14.    An apparatus for non-invasive uterine insertion to measure light extinction during transillumination of a portion of blood-perfused fetal tissue, comprising:

an introducing and orienting probe having a surface for fetal cutaneous conformance;

a light source mounted on said probe surface to emit light to and transilluminate said fetal tissue;

a light sensor mounted on said probe surface to receive light having transilluminated said fetal tissue; and

detachable, semiflexible columnar tubing associated with said probe.

-23-

15.    The apparatus of claim 14, wherein a needle screw is provided on the probe for attachment of the probe to said fetal tissue.

16.    The apparatus of claim 14 or 15, wherein suction means are provided for attachment of the probe to said fetal tissue.

17.    An apparatus for non-invasive uterine insertion to measure light extinction during transillumination of a portion of blood-perfused fetal tissue, comprising:

an introducing and orienting probe having a surface for fetal cutaneous conformance;

a light source mounted on said probe surface to emit light to and transilluminate said fetal tissue;

a light sensor mounted on said probe surface to receive light having transilluminated said fetal tissue; and

adhesive means for attachment of the probe to said fetal tissue.

18.    An apparatus for non-invasive uterine insertion to measure light extinction during transillumination of a portion of blood-perfused fetal tissue and of a portion of uterine lining comprising:

an introducing and orienting probe means having a first side for fetal conformance and a second side for uterine conformance;

a light source mounted on each said probe side to emit light to and through said respective fetal tissue or uterine lining;

a light sensor mounted on each said probe side to receive light having transilluminated said respective fetal tissue or uterine lining, and

rounded protrusions on said first probe side to provide frictional gripping between said probe and said fetal tissue and to aid in separating any hair thereon to expose the fetal tissue.

19. An apparatus for non-invasive uterine insertion to measure light extinction during transillumination of a portion of blood-perfused fetal tissue and of a portion of uterine lining comprising:

an introducing and orienting probe means having a first side for fetal conformance and a second side for uterine conformance;

a light source mounted on each said probe side to emit light to and through said respective fetal tissue or uterine lining; and

a light sensor mounted on each said probe side to receive light having transilluminated said respective fetal tissue or uterine lining, each said light sensor being recessed into its respective probe side.

20. An apparatus for non-invasive uterine insertion to measure light extinction during transillumination of a presenting portion of blood-perfused fetal tissue, comprising:

a suction cup probe device having a concave surface for contacting and suctionally securing said probe to said fetal tissue;

a light source mounted on the concave surface of said probe for emitting light to transilluminate said fetal tissue; and

a light sensor mounted on the concave surface of said probe for receiving the light trans-illuminating said fetal tissue.

FIG _ 1.

FIG _ 2.

FIG. 9

FIG._3.

FIG._4.    FIG._5

FIG._6A.

FIG._6B.

FIG._7

FIG._8

FIG._10

FIG._11

FIG._12

FIG._13

FIG._16

4/5

0135840

FIG.__14

FIG.__15

5/5

0135840